# EUROPÄISCHE PATENTSCHRIFT

(11) **EP 0 788 477 B1**
(45) Veröffentlichungstag und Bekanntmachung des Hinweises auf die Patenterteilung: **26.01.2000**
(21) Anmeldenummer: 95937835.7
(22) Anmeldetag: 24.10.1995
(51) Int. Cl.: C07C 409/22

(54) **HERSTELLUNG VON SALZFREIEM UND WASSERARMEM METHYLETHYLKETONPEROXID**
PRODUCTION OF SALT-FREE AND LOW-WATER-CONTENT METHYL ETHYL KETONE PEROXIDE
PREPARATION DE PEROXYDE DE METHYL ETHYL CETONE EXEMPT DE SEL ET A FAIBLE TENEUR EN EAU

(30) Priorität: 25.10.1994 DE 4438147
(43) Veröffentlichungstag der Anmeldung: 13.08.1997
(73) Patentinhaber: Peroxid-Chemie GmbH & Co. KG., 82049 Pullach (DE)
(72) Erfinder: DORN, Maximilian, D-82031 Grünwald (DE); ZIMMERMANN, Heinz, D-81477 München (DE); HÄGEL, Eberhard, D-82057 Icking (DE)
(74) Vertreter: Böhm, Brigitte, Dipl.-Chem. Dr.
(86) Internationale Anmeldenummer: EP9504170
(87) Internationale Veröffentlichungsnummer: WO9612698

(56) Entgegenhaltungen:
- DE-A- 2 952 667
- FR-A- 1 551 955
- GB-A- 912 061
- GB-A- 944 873

## Beschreibung

Die Erfindung betrifft ein Verfahren zur Herstellung von Methylethylketonperoxid aus Methylethylketon und Wasserstoffperoxid.

Methylethylketonperoxid (MEKP; 2-Butanon-peroxid) wird insbesondere als Polymerisationskatalysator vor allem zur Kalthärtung von ungesättigten Polyesterharzen, z.B. bei der Herstellung von Polyesterbooten, verwendet. Üblicherweise liegt es als 30 bis 50 %-ige Lösung in Phlegmatisierungsmitteln vor.

Herstellungsverfahren für Methylethylketonperoxide (MEKP) aus Methylethylketon (MEK) und Wasserstoffperoxid sind bekannt. Die DE-AS-1243195 beschreibt beispielsweise ein Verfahren zur Herstellung von homogenen wasserhaltigen Lösungen von niedermolekularen Ketonperoxiden, z.B. von Methylethylketonperoxid, durch Umsetzung eines Ketons unterhalb 30°C in saurer Umgebung mit 60 %igem Wasserstoffperoxid, wobei die Umsetzung in einem inerten hydrophilen organischen Lösungsmittel und/oder in Gegenwart eines heterozyklischen Amides durchgeführt wird. Hierbei wird ein Produkt erhalten, das 16 % Wasser enthält, was einen entscheidenden Nachteil darstellt.

Die JP-A-48023709 beschreibt die Herstellung von Methylethylketonperoxid durch Umsetzung von Methylethylketon und Wasserstoffperoxid in Dimethylformamid in Gegenwart von p-Toluolsulfonsäure.

Die JP-B-70019486 beschreibt die Herstellung nicht explosiver Ketonperoxide, z.B. von Methylethylketonperoxid, durch Umsetzung eines Ketons, z.B. von Methylethylketon, mit Wasserstoffperoxid in Gegenwart einen Säurekatalysators und eines Phosphorsäureesters, und Behandlung des Reaktionsproduktes mit einem oder mehreren Glykolen.

Die DE-C-2952667 beschreibt die Herstellung eines Gemisches zweier Ketonperoxide in einem Zweistufenverfahren, bei dem in einer ersten Stufe Methylethylketonperoxid durch Peroxidierung hergestellt wird, wobei in dieser Stufe Salze zugegeben werden und dann in einer zweiten Stufe ein weiteres Ketonperoxid im gleichen Reaktionsansatz hergestellt wird. Aus der ersten Verfahrensstufe enthält das entstehende Ketonperoxidgemisch jedoch Salz, was, wie weiter unten ausgeführt wird, einen erheblichen Nachteil darstellt.

Nach den gängigen Verfahren zur Herstellung von MEKP-Lösungen werden entweder Zweiphasen-MEKP-Lösungen oder Einphasen-MEKP-Lösungen erhalten.

In einem typischen Verfahren zur Herstellung von Zweiphasen-MEKP-Lösungen wird zu einer Lösung von Methylethylketon in einem mit Wasser nicht mischbaren Phlegmatisierungsmittel ein Säurekatalysator, z.B. Mineralsäure, Schwefelsäure, Salpetersäure, Phosphorsäure, zugegeben, und dann unter Rühren und Kühlen Wasserstoffperoxid einer Konzentration von 35 bis 70 % zugegeben; nach erfolgter Umsetzung wird die Mischung in eine wäßrige und eine organische Phase getrennt, wobei zur besseren Phasentrennung und zur Vermeidung größerer Verluste an Wasserstoffperoxid und wasserlöslichen MEKP-Typen die wäßrige Phase mit Salzen beschwert wird, d.h. der wäßrigen Phase Salze, z.B. Na₂SO₄, zugesetzt werden. Die organische Phase wird dann nach Abpuffern und Trocknen filtriert. Ein derartiges Verfahren ist in der DE-PS 832743 beschrieben.

Obwohl nach diesem Verfahren ein Wasserstoffperoxid mit einer Konzentration bis zu 70 % verwendet wird, also handelsübliche Konzentrationen, die sicher handhabbar sind, und durch die Trocknungsstufe ein niedriger Wassergehalt (ca. 2.5 bis 3.5 %) erreichbar ist, besitzt dieses Verfahren aber den Nachteil, daß ein großer Teil des eingesetzten Aktiv-Sauerstoffs, nämlich ca. 25 %, bei der Umsetzung verloren gehen, und aufgrund der Verwendung von Trocknungsmitteln hohe Produktverluste und hohe Abfallmengen, und damit Umweltprobleme, entstehen; durch den Zusatz von Salzen zur besseren Phasentrennung enthält das Methylethylketonperoxid außerdem auch noch Spuren von gelösten Salzen, die zu Kristallabscheidungen führen können; das als Phlegmatisierungsmittel in diesem Verfahren üblicherweise verwendete Dimethylphthalat ist außerdem teuer.

Nach dem Verfahren zur Herstellung von Einphasen-MEKP-Lösungen wird zu einer Lösung von Methylethylketon in einem mit Wasser mischbaren Lösungsmittel oder einer Mischung von in Wasser mischbaren und nicht-mischbaren Lösungsmitteln Säurekatalysator, z.B. HNO₃, zugegeben und unter Rühren und Kühlen 85 %-iges H₂O₂ zudosiert; eine Phasentrennung, Trocknung und Filtration erfolgt nach diesem Verfahren nicht.

Ein Vorteil dieses Verfahrens ist es, daß praktisch kein Verlust an Aktiv-Sauerstoff auftritt und keine Abfälle und die damit verbundene Umweltverschmutzung auftreten. Da auch keine Filtration erfolgt, und deshalb kein Filter erforderlich ist, ist der apparative Aufwand geringer als beim Verfahren zur Herstellung von Zweiphasen-MEKP-Lösungen. Außerdem sind im erhaltenen Methylethylketonperoxid-Endprodukt keine Salze vorhanden, und es ist der Einsatz preisgünstiger Phlegmatisierungsmittel, z.B. von Diacetonalkohol in Mischung mit Dibutylphthalat, möglich. Derartige Verfahren sind in der DE-PS 12 43 195, welche oben bereits erwähnt wurde, und in der DE-PS 12 89 047 beschrieben.

Ein großer Nachteil dieses Verfahrens ist es aber, daß, wenn mit einem 85 % -igen H₂O₂ gearbeitet wird, das, abgesehen davon, daß es nicht überall erhältlich ist, teuer ist und seine Handhabung unter bestimmten Vorsichtsmaßnahmen erfolgen muß. Außerdem wird ein Produkt mit einem relativ hohen Wassergehalt (7 bis 8 %) erhalten. Bei Verfahren mit 60 %igem H₂O₂ steigt der Wassergehalt des Produkts bis auf 16 %. Der hohe Wassergehalt kann zu Qualitätseinbußen bei den damit gehärteten UP-Harz-Teilen führen.

Aufgabenstellung der vorliegenden Erfindung war es, ein Verfahren zur Herstellung von Methylethylketonperoxid bereitzustellen, das salzfrei und wasserarm ist und damit die vorstehend genannten Nachteile weitgehend vermieden werden können, und mit dem auf einfache und kostengünstige Weise Methylethylketonperoxid-Zusammensetzungen erhalten werden können.

Diese Aufgabenstellung wird mit der vorliegenden Erfindung gelöst.

Gegenstand der Erfindung ist ein Verfahren gemäß Patentanspruch 1 zur Herstellung von Methylethylketonperoxid durch Umsetzung von Methylethylketon und Wasserstoffperoxid in Gegenwart eines Säurekatalysators in einem Phlegmatisierungsmittel, das dadurch gekennzeichnet ist, daß man als Wasserstoffperoxid ein 50 bis 70 %-iges Wasserstoffperoxid einsetzt, die wäßrige Phase aus dem nach der Umsetzung erhaltenen Zweiphasensystem ohne Salzzugabe abtrennt, gegebenenfalls mit geringen Mengen von organischen oder anorganischen Basen (z.B. Collidin oder Natriumhydrogencarbonat) die restlichen Säurespuren neutralisiert und zur organischen Phase soviel eines mit Wasser mischbaren Phlegmatisierungsmittel zugibt, daß eine klare Methylethylketonperoxidlösung erhalten wird.

Zweckmäßige Ausgestaltungen des Verfahrens sind Gegenstand der Ansprüche 2 bis 6.

Mit dem erfindungsgemäßen Verfahren läßt sich durch Verwendung von handelsüblichem H₂O₂ und Verwendung billiger Phlegmatisierungsmittel das Methylethylketonperoxid auf sichere und kostengünstige Weise erhalten. Obwohl im erfindungsgemäßen Verfahren ein Zweiphasengemisch auftritt, werden erfindungsgemäß keine Salze zugegeben, wodurch das Auftreten von Salzen im Endprodukt oder als Abfall vermieden werden kann; da außerdem keine Filter benötigt werden, kann das Verfahren mit einem geringen apparativen Aufwand durchgeführt werden. Der Wassergehalt der erhaltenen MEKP-Lösung liegt mit 4 bis 5 % außerdem niedriger als bei den nach dem vorstehend beschriebenen Einphasen-Verfahren unter Verwendung von 85 %-igem H₂O₂ erhaltenen MEKP-Lösungen.

Das Produkt des erfindungsgemäßen Verfahrens ist daher ein salzfreies und wasserarmes MEKP, welches mit preiswerten Lösungsmitteln und niedrig konzentriertem Wasserstoffperoxid hergestellt wird.

Der Zusatz an mit Wasser mischbaren Phlegmatisierungsmitteln, z.B. Diacetonalkohol, Diethylenglykol, Dipropylenglykol, Alkylglykolether, Triethylphosphat, zur organischen Phase erfolgt vorzugsweise in einer solchen Menge, daß die Methylethylketonperoxid-Lösung auch bei tiefen Lagertemperaturen, wie z.B. bei Raumtemperatur oder darunter, klar und lagerbeständigkeit bleibt.

Vorzugsweise wird im erfindungsgemäßen Verfahren zusätzlich ein nicht mit Wasser mischbares Phlegmatisierungsmittel verwendet. Als solche Phlegmatisierungsmittel werden erfindungsgemäß Di-n-amylphthalat, Butylbenzylphthalat, Dioctylphthalat, Dibutylphthalat (DBP), Diisobutylphthalat (DIBP) oder Gemische davon verwendet. Bei diesen Lösungsmitteln handelt es sich um leicht erhältliche und relativ billige Produkte, wodurch das erfindungsgemäße Verfahren kostengünstig durchzuführen ist.

Zur organischen Phase wird mit dem Phlegmatisierungsmittel vorzugsweise noch zusätzliches 50 bis 70 %-iges Wasserstoffperoxid zugegeben.

Das erfindungsgemäße Verfahren wird vorzugsweise bei einer Temperatur im Bereich von 20 bis 50°C, und insbesondere im Bereich von 25 bis 35°C durchgeführt.

Als Säurekatalysator kann ein für die Umsetzung von Alkylketonen mit Wasserstoffperoxid üblicherweise verwendeter Säurekatalysator eingesetzt werden, wie z.B. Mineralsäuren; erfindungsgemäß wird vorzugsweise mit Schwefelsäure, und/oder Phosphorsäure, und insbesondere mit Salpetersäure als Säurekatalysator gearbeitet. Die zugesetzte Menge an Säurekatalysator entspricht der für diese Umsetzungen üblichen Menge.

Zur Verbesserung der Lagerstabilität der erfindungsgemäß erhaltenen MEKP-Lösungen können Stabilisatoren (z.B. Komplexbildner für Schwermetalle, pH-Stabilisatoren u.s.w.) zugesetzt werden.

Durch Auswahl geeigneter Reaktionsbedingungen, insbesondere durch Wahl des Lösungsmittels (mehr hydrophil oder hydrophob), der Katalysatormenge, und des Molverhältnisses von Methylethylketon zu Wasserstoffperoxid, läßt sich die Zusammensetzung (Speciesverteilung der in der Methylethylketonperoxid-Lösung enthaltenen Methylethylketonperoxid-Typen) in weiten Grenzen variieren.

Das nachfolgende Beispiel soll nun die Erfindung näher erläutern, ohne sie darauf zu beschränken.

### Beispiel

In einem 2 Liter-Glasreaktor werden Dibutylphthalat (600 g, 577 ml), Methylethylketon (454 g, 564 ml) und 65 %-ige HNO₃ (1.4 g, 1 ml) vorgelegt. Dazu werden während ca. 1 Stunde unter Rühren und Kühlen 474 ml (612 g) 70 %-iges Wasserstoffperoxid so zugegeben, daß die Temperatur der Reaktionsmischung auf 30°C gehalten wird. Nach Beendigung der Zugabe wird dann noch 30 Minuten bei 30°C nachgerührt, der Rührer dann abgestellt und nach 10 Minuten wird die wäßrige Phase (396 g, 375 ml) abgetrennt. Zur organischen Phase werden dann unter Rühren Diacetonalkohol (200 g, 250 ml), Collidin (2.8 g, 3 ml) und 70 %-iges Wasserstoffperoxid (24 g, 19 ml) zugegeben und danach noch 5 Minuten lang gerührt.

Es wird eine salzfreie klare farblose Lösung erhalten. Die Ausbeute an aktivem Sauerstoff beträgt ca. 70 %; der Wassergehalt der Lösung beträgt ca. 4.5 %.

Die Dichte (d²⁰, g/cm³) beträgt 1.05.

## Patentansprüche

1. Verfahren zur Herstellung von Methylethylketonperoxid durch Umsetzung von Methylethylketon und Wasserstoffperoxid in Gegenwart eines Säurekatalysators in einem Phlegmatisierungsmittel,
**dadurch gekennzeichnet,**
daß man als Wasserstoffperoxid ein 50 bis 70 %-iges Wasserstoffperoxid einsetzt, die wäßrige Phase aus dem nach der Umsetzung erhaltenen Zweiphasengemisch ohne Salzzugabe abtrennt und zur organischen Phase so viel eines mit Wasser mischbaren Phlegmatisierungsmittels zugibt, daß eine klare Methylethylketonperoxid-Lösung erhalten wird.

2. Verfahren nach Anspruch 1,
**dadurch gekennzeichnet,**
daß man zur organischen Phase mit dem Phlegmatisierungsmittel noch zusätzliches 50 bis 70 %-iges Wasserstoffperoxid zugibt.

3. Verfahren nach Anspruch 1 oder 2,
**dadurch gekennzeichnet,**
daß man bei einer Temperatur im Bereich von 20 bis 50°C, insbesondere von 25 bis 35°C, arbeitet.

4. Verfahren nach einem der Ansprüche 1 bis 3,
**dadurch gekennzeichnet,**
daß man als Phlegmatisierungsmittel zusätzlich ein mit Wasser nicht mischbares Phlegmatisierungsmittel verwendet.

5. Verfahren nach einem der Ansprüche 1 bis 4,
**dadurch gekennzeichnet,**
daß man als Säurekatalysator Salpetersäure verwendet.

6. Verfahren nach einem der vorhergehenden Ansprüche,
**dadurch gekennzeichnet,**
daß man nach Abtrennung der wäßrigen Phase gegebenenfalls noch vorhandene Säurespuren mit geringen Mengen von organischen oder anorganischen Basen neutralisiert.

## Claims

1. Process for the production of methyl ethyl ketone peroxide by reacting methyl ethyl ketone and hydrogen peroxide in the presence of an acid catalyst in a desensitization agent,
**wherein**
a 50 to 70 % hydrogen peroxide is used as the hydrogen peroxide, the aqueous phase is separated from the two-phase system obtained after the reaction without addition of a salt and as much of a water-miscible desensitization agent is added to the organic phase as is required to obtain a clear methyl ethyl ketone peroxide solution.

2. Process as claimed in claim 1,
**wherein**
50 to 70 % hydrogen peroxide is additionally added to the organic phase with the desensitization agent.

3. Process as claimed in claim 1 or 2,
**wherein**
one works at a temperature in the range of 20 to 50°C, in particular of 25 to 35°C.

4. Process as claimed in one of the claims 1 to 3,
**wherein**
a desensitization agent that is immiscible with water is additionally used as the desensitization agent.

5. Process as claimed in one of the claims 1 to 4,
**wherein**
nitric acid is used as an acid catalyst.

6. Process as claimed in one of the previous claims,
**wherein**
after separation of the aqueous phase traces of acid that may still be present are neutralized with small amounts of organic or inorganic bases.

## Revendications

1. Procédé pour la préparation de peroxyde de méthyléthylcétone par mise en réaction de méthyléthylcétone et de peroxyde d'hydrogène en présence d'un catalyseur acide dans un milieu de stabilisation,
caractérisé en ce qu'en tant que peroxyde d'hydrogène on met en oeuvre un peroxyde d'hydrogène à 50 jusqu'à 70%, en ce que l'on sépare la phase aqueuse du mélange à deux phases obtenues après la réaction sans addition de sel et en ce que pour la phase organique on ajoute une quantité telle de milieu de stabilisation miscible dans l'eau pour obtenir une solution de peroxyde de méthyléthylcétone limpide.

2. Procédé selon la revendication 1, caractérisé en ce que pour la phase organique avec le milieu de stabilisation on ajoute encore du peroxyde d'hydrogène supplémentaire à 50 jusqu'à 70%.

3. Procédé selon la revendication 1 ou 2, caractérisé en ce que l'on travaille à une température dans la plage de 20 à 50°C, en particulier de 25 à 35°C.

4. Procédé selon l'une des revendications 1 à 3, caractérisé en ce qu'en tant que milieu de stabilisation on utilise de plus un agent de stabilisation non miscible dans l'eau.

5. Procédé selon l'une des revendications 1 à 4, caractérisé en ce qu'en tant que catalyseur acide on utilise de l'acide nitrique.

6. Procédé selon l'une des revendications précédentes, caractérisé en ce que après la séparation de la phase aqueuse on neutralise éventuellement les traces d'acide encore présentes avec de petites quantités de base organique ou minérale.
